(19) Europäisches Patentamt
European Patent Office
Office européen des brevets

(11) Veröffentlichungsnummer: **0 481 351 A2**

# (12) EUROPÄISCHE PATENTANMELDUNG

(21) Anmeldenummer: **91117253.4**

(51) Int. Cl.[5]: **C07D 201/08**, C07D 207/267

(22) Anmeldetag: **10.10.91**

(30) Priorität: **19.10.90 DE 4033259**

(43) Veröffentlichungstag der Anmeldung: **22.04.92 Patentblatt 92/17**

(84) Benannte Vertragsstaaten: **BE CH DE ES FR GB IT LI NL**

(71) Anmelder: **BASF Aktiengesellschaft**
**Carl-Bosch-Strasse 38**
**W-6700 Ludwigshafen(DE)**

(72) Erfinder: **Schuster, Ludwig, Dr.**
**Weinheimer Strasse 44**
**W-6703 Limburgerhof(DE)**
Erfinder: **Koehler, Ulrich, Dr.**
**Werderstrasse 48**
**W-6900 Heidelberg(DE)**

## (54) Verfahren zur Herstellung von 2-Pyrrolidinonen.

(57) Verfahren zur Herstellung von 2-Pyrrolidinonen der allgemeinen Formel I

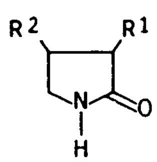

(I),

in der die Substituenten

$R^1$ und $R^2$ unabhängig voneinander Wasserstoff, $C_1$- bis $C_{20}$-Alkyl, oder gegebenenfalls durch $C_1$- bis $C_4$-Alkyl substituiertes $C_3$- bis $C_8$-Cycloalkyl oder Phenyl bedeuten,

durch Umsetzung von 3-Cyanopropionsäureester der allgemeinen Formel II

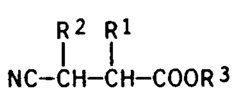

(II),

in der $R^1$ und $R^2$ die obengenannten Bedeutungen haben und $R^3$ für $C_1$- bis $C_8$-Alkyl steht, mit Wasserstoff bei erhöhten Temperaturen und erhöhtem Druck, indem man die Umsetzung in Gegenwart von Ammoniak an einem Katalysator, der Cobalt, Mangan und Phosphor enthält, durchführt.

EP 0 481 351 A2

Die vorliegende Erfindung betrifft ein neues und verbessertes Verfahren zur Herstellung von 2-Pyrrolidinonen durch Hydrierung von 3-Cyanopropionsäureestern in Gegenwart von Ammoniak an einem Katalysator, der Cobalt, Mangan und Phosphor enthält.

An der DE-A-16 70 575 ist ein verfahren zur Herstellung von Pyrrolidinonen durch drucklose Gasphasenhydrierung von 3-Cyanopropionsäureester an Festbettkatalysatoren bekannt, die aus Raneymetallen gegebenenfalls mit Chromoxid, aus Edelmetallen bzw. deren Mischkatalysatoren mit Aluminiumoxid bestehen. Nachteilig an diesem Verfahren ist das ungünstige verhältnis von umgewälztem Wasserstoff zum Produkt.

Aus der EP-A-23 751 ist die Hydrierung von β-Cyanopropionsäuremethylester an einem Rutheniumkatalysator bekannt, wobei die Ausbeute an Pyrrolidinon zu wünschen übrig läßt.

Der vorliegenden Erfindung lag daher die Aufgabe zugrunde, den zuvor genannten Nachteilen abzuhelfen.

Demgemäß wurde ein neues und verbessertes Verfahren zur Herstellung von 2-Pyrrolidinonen der allgemeinen Formel I

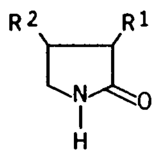

(I),

in der

$R^1$ und $R^2$ unabhängig voneinander Wasserstoff, $C_1$- bis $C_{20}$-Alkyl, oder gegebenenfalls durch $C_1$- bis $C_4$-Alkyl substituiertes $C_3$- bis $C_8$-Cycloalkyl oder Phenyl bedeuten,

durch Umsetzung von 3-Cyanopropionsäureester der allgemeinen Formel II

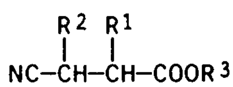

(II),

in der $R^1$ und $R^2$ die obengenannten Bedeutungen haben und $R^3$ fur $C_1$- bis $C_8$-Alkyl steht, mit Wasserstoff bei erhöhten Temperaturen und erhöhtem Druck gefunden, welches dadurch gekennzeichnet ist, daß man die Umsetzung in Gegenwart von Ammoniak an einem Katalysator, der Cobalt, Mangan und Phosphor enthält, durchführt.

Das erfindungsgemäße Verfahren zur Herstellung der 2-Pyrrolidinone I kann wie folgt durchgeführt werden:

3-Cyanopropionsäureester II und Wasserstoff werden in Gegenwart von Ammoniak an einem Katalysator, der Cobalt, Mangan und Phosphor enthält bei erhöhten Temperaturen und Drücken diskontinuierlich, insbesondere aber kontinuierlich, in einem oder mehreren Reaktoren Umgesetzt. Man kann in Sumpf-, insbesondere aber Rieselfahrweise arbeiten, wobei der Katalysator fest angeordnet ist. Es ist aber auch möglich, einen suspendierten Katalysator einzusetzen. Man kann sowohl Wasserstoff als auch einen Teil des Hydrieraustrags in die Hydrierstufe zurückleiten.

Als Reaktoren können z.B. Rohrreaktoren oder Rohrbündelreaktoren verwendet werden, wobei die Reaktionswärme durch Außenkühlung oder im Fall von Rohrreaktoren durch Innenkühlung abgeführt werden kann. Vorzugsweise verwendet man Rieseltürme mit fest angeordneten Katalysatorteilchen in Form von Pillen, strängen, Kugeln oder anderen Körpern, die eine regelmäßige Schüttung ermöglichen.

Hierbei kann es sich als günstig erweisen, die Reaktion im Kreis zu führen, wobei ein Teil des ausreagierten Produktes, mit Ausgangslösung vermischt, wieder auf den Kopf des Reaktionsturmes zurückgeführt wird.

Die Hydrierung wird im allgemeinen bei Temperaturen von 50 bis 200 ° C, insbesondere 70 bis 100 ° C, durchgeführt. Der Reaktionsdruck kann innerhalb weiter Grenzen schwanken. Man arbeitet bei Drücken von 2 bis 500 bar, bevorzugt bei 50 bis 300 bar, besonders bevorzugt bei 200 bis 300 bar.

Um die hohe Hydrierwärme ohne Schädigung des Katalysators abzuführen, kann es sinnvoll sein, die Hydrierung auf zwei verschiedenen Temperatur- und/oder Druckniveaus durchzuführen. Eine Aufarbeitung nach dem ersten Temperatur-/Druckniveau erfolgt nicht. So kann man z.B. zunächst bei Temperaturen von 50 bis 130 ° C und Drücken von 50 bis 100 bar arbeiten, um dann die Hydrierung bei Temperaturen von 90

bis 200 °C und Drücken von 200 bis 300 bar Zu vervollständigen.

Erfindungsgemäß werden zur katalytischen Hydrierung der Reaktionsmischungen Katalysatoren verwendet, die Cobalt, Mangan und Phosphor enthalten. Bevorzugt werden Katalysatoren benutzt, welche neben Cobalt, Mangan und Phosphor auch noch Alkali enthalten. Solche Katalysatoren sowie ihre Herstellung sind in DE-A-23 01 139, FR-A-18 43 299 und FR-A-14 83 300 beschrieben.

Vorteilhaft können im erfindungsgemäßen Verfahren beispielsweise solche Katalysatoren angewandt werden, deren katalytisch aktive Masse im nicht reduzierten Zustand mindestens 60 Gew.-% CoO vorzugsweise 70 bis 95 Gew.-% CoO enthält. Als weitere aktive Bestandteile können 2 bis 10 Gew.-%, vorzugsweise 3 bis 7 Gew.-% $Mn_3O_4$, 0,5 bis 10 Gew.-%, vorzugsweise 1 bis 5 Gew.-% Phosphorsäure und 0 bis 2 Gew.-%, vorzugsweise 0,01 bis 0,8 Gew.-% Alkalioxid, bevorzugt $Na_2O$, enthalten sein.

Die erfindungsgemäß verwendeten Katalysatoren können sowohl als Trägerkatalysatoren oder vorzugsweise in kompakter Form, d.h. ohne Träger eingesetzt werden. Die Art des Trägermaterials ist in der Regel nicht kritisch, es können übliche Trägermaterialien wie Siliciumdioxid, Aluminiumoxide, Titandioxide, Aktivkohle, Silikate oder Zeolithe verwendet werden. Erforderlichenfalls können zur Herstellung der Katalysatoren Bindemittel oder Formhilfsmittel mitverwendet werden.

Die Katalysatoren werden vorzugsweise vor ihrem Einsatz im erfindungsgemäßen Verfahren mit Wasserstoff aktiviert. Dabei werden die nach der Calcinierung im allgemeinen in Form ihrer Oxide vorliegenden aktiven Katalysatorbestandteile größtenteils reduziert und zwar in der Regel zu den entsprechenden Metallen. Weitere Einzelheiten zur Herstellung dieser Katalysatoren können DE-A 23 01 139 sowie der FR-A-18 43 299 und der FR-A-14 83 300 entnommen werden.

Die Katalysatoren können im erfindungsgemäßen verfahren in suspendierter Form angewandt werden, bevorzugt ist jedoch eine Festbettanordnung des Katalysators, über die die Einsatzstoffe in der Sumpf- oder vorzugsweise der Rieselfahrweise geleitet werden.

Die erfindungsgemäße Hydrierung kann in Gegenwart eines Lösungsmittels durchgeführt werden. Als Lösungsmittel kommen aliphatische und cyclische Ether, z.B. Diethylether, Methyl-tert.-butylether, Dioxan, Tetrahydrofuran und/oder die Reaktionsprodukte oder z.B. N-Methylpyrrolidon, besonders bevorzugt Wasser, in Betracht.

Das Molverhältnis von den Lösungsmitteln zum 3-Cyanopropionsäureester und Ammoniak beträgt 0,001 : 1 bis 100 : 1, insbesondere 5 : 1 bis 50 : 1. Die Reaktion kann jedoch auch bevorzugt lösungsmittelfrei durchgeführt werden.

Das Molverhältnis von Ammoniak zum 3-Cyanopropionsäureester II beträgt im allgemeinen 1 : 1 bis 50 : 1, vorzugsweise 1 : 1 bis 20 : 1, besonders bevorzugt 1 : 1 bis 5 : 1.

Die Aufarbeitung des Hydrieraustrages kann durch Destillation und/oder Extraktion erfolgen.

Die Substituenten $R^1$, $R^2$ und $R^3$ in den verbindungen I und II haben folgende Bedeutungen:

$R^1$, $R^2$ unabhängig voneinander

- Wasserstoff
- $C_1$- bis $C_{20}$-Alkyl, bevorzugt $C_1$- bis $C_8$-Alkyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl,
- $C_3$- bis $C_8$-Cycloalkyl, wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- Phenyl
- durch $C_1$- bis $C_4$-Alkyl ein- bis dreifach substituiertes $C_3$- bis $C_8$-Cycloalkyl,
- durch $C_1$- bis $C_4$-Alkyl ein- bis dreifach substituiertes Phenyl,

$R^3$

- $C_1$- bis $C_8$-Alkyl, bevorzugt $C_1$- bis $C_4$-Alkyl wie Methyl, Ethyl, n-Propyl, iso-Propyl, n-Butyl, iso-Butyl, sec.-Butyl und tert.-Butyl, besonders bevorzugt Methyl und Ethyl.

Besonders bevorzugt ist Pyrrolidinon, in dem die Reste $R^1$ und $R^2$ jeweils Wasserstoff bedeuten.

Die Pyrrolidinone I und speziell Pyrrolidinon ($R^1$, $R^2$ = H) eignen sich als Lösungsmittel und als Ausgangsstoffe für weitere Synthesen.

## Beispiele

### Beispiel 1

100 g 3-Cyanopropionsäuremethylester, 300 ml Tetrahydrofuran und 30 g Katalysator, im nichtreduzierten Zustand bestehend aus: 80,5 Gew.-% CoO, 5 Gew.-% $Mn_3O_4$, 3,28 Gew.-% $H_3PO_4$ und 0,22 Gew.-% $Na_2O$ wurden in einem 1,2 1 Autoklaven mit 100 g Ammoniak zusammengegeben und anschließend soviel

Wasserstoff aufgepreßt, daß ein Druck von 60 bar entstand. Der Autoklav wurde innerhalb von 15 min auf 130 °C hochgeheizt. Nach Erreichen dieser Temperatur wurde der Druck mit Wasserstoff auf 300 bar erhöht. Innerhalb von 15 min fiel der Druck auf 80 bar ab, worauf die Reaktion beendet war. Nach dem Entspannen filtrierte man vom Katalysator ab und destillierte fraktioniert. Man erhielt 74,6 g (99,2 %) Pyrrolidinon; Sdp. 95 bis 100 °C (0,1 mbar).

Beispiel 2

In einem Reaktor von 2 m Länge und 45 mm Durchmesser wurden 2,7 l eines Katalysators eingefüllt, der aus einem Granulat von 4 mm Durchmesser bestand und die gleiche Zusammensetzung aufwies wie in Beispiel 1. Der Katalysator wurde im Reaktor durch langsames Erhitzen auf 350 °C in einem Gasstrom von 10 vol% Wasserstoff und 90 vol% Stickstoff erhitzt. Nach dem Erreichen von 350 °C wurde im verlauf von 24 Stunden der Wasserstoffgehalt des Reduktionsgases auf 100 % erhöht.

Anschließend wurden stündlich 3,25 kg einer 25-prozentigen Lösung von 3- Cyanopropionsäuremethylester in Tetrahydrofuran gleichzeitig mit Ammoniak zugepumpt, wobei das molare verhältnis von Ammoniak zu Cyanopropionsäuremethylester 5:1 war. Die Temperatur in der Säule betrug 130 °C; der Druck lag bei 300 bar. Wasserstoff wurde auf den Kopf der Kontaktsäule eingespeist. Zur Rückführung von Reaktionsprodukt wurde dieses in einer Menge von 50 $m^3/m^2 \cdot h$ im Umlauf bewegt. Bei vollständigem Umsatz erhielt man eine Ausbeute von 94,5 %.

Beispiel 3

Die Reaktion wurde in derselben Apparatur wie im Beispiel 2 durchgeführt, jedoch wurden 1,6 kg 3-Cyanopropionsäuremethylester mit der fünffachen molaren Menge Ammoniak pro Stunde zugepumpt. Die zurückgeführte Menge war so groß, daß wieder eine Querschnittsbelastung von 50 $m^3/m^2 \cdot h$ erreicht wurde. Bei quantitativem Umsatz erhielt man eine Ausbeute von 90 %.

**Patentansprüche**

**1.** Verfahren zur Herstellung von 2-Pyrrolidinonen der allgemeinen Formel I

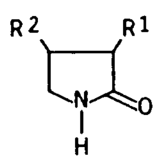

(I),

in der die Substituenten

$R^1$ und $R^2$ unabhängig voneinander Wasserstoff, $C_1$- bis $C_{20}$-Alkyl, oder gegebenenfalls durch $C_1$- bis $C_4$-Alkyl substituiertes $C_3$- bis $C_8$-Cycloalkyl oder Phenyl bedeuten,

durch Umsetzung von 3-Cyanopropionsäureester der allgemeinen Formel II

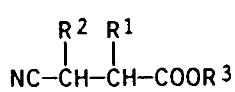

(II),

in der $R^1$ und $R^2$ die obengenannten Bedeutungen haben und $R^3$ für $C_1$- bis $C_8$-Alkyl steht, mit Wasserstoff bei erhöhten Temperaturen und erhöhtem Druck, dadurch gekennzeichnet, daß man die Umsetzung in Gegenwart von Ammoniak an einem Katalysator, der Cobalt, Mangan und Phosphor enthält, durchführt.

**2.** Verfahren nach Anspruch 1, dadurch gekennzeichnet, daß die aktive Masse des Katalysators im nicht reduzierten Zustand mindestens zu 60 Gew.-% CoO enthält.

**3.** Verfahren nach Anspruch 1, dadurch gekennzeichnet, daß man einen Katalysator verwendet, dessen aktive Masse im nicht reduzierten Zustand zu 70 bis 95 Gew.-% CoO, zu 2 bis 15 Gew.-% $Mn_3O_4$, zu

0,5 bis 10 Gew.-% $H_3PO_4$ und zu 0 bis 2 Gew.-% Alkalioxid enthält.

4. Verfahren nach Anspruch 1, dadurch gekennzeichnet, daß man bei Temperaturen von 50 bis 200 °C arbeitet.

5. Verfahren nach Anspruch 1, dadurch gekennzeichnet, daß man bei Drücken von 2 bis 500 bar arbeitet.

6. Verfahren nach Anspruch 1, dadurch gekennzeichnet, daß man ein Molverhältnis von Ammoniak zum 3-Cyanopropionsäureester II von 1 : 1 bis 50 : 1 einhält.

7. Verfahren nach Anspruch 1, dadurch gekennzeichnet, daß man ein Molverhältnis von Ammoniak zum 3-Cyanopropionsäureester II von 1 : 1 bis 20 : 1 einhält.

8. Verfahren nach Anspruch 1, dadurch gekennzeichnet, daß $R^1$ und $R^2$ jeweils Wasserstoff bedeuten.